# NEUE EUROPÄISCHE PATENTSCHRIFT

(11) **EP 2 453 973 B2**
(45) Veröffentlichungstag und Bekanntmachung des Hinweises auf die Entscheidung über den Einspruch: **28.03.2018**
(45) Hinweis auf die Patenterteilung: 13.05.2015
(21) Anmeldenummer: 11745723.4
(22) Anmeldetag: 15.07.2011
(51) Int. Cl.: A61M 39/10

(54) **VERBINDUNGSVORRICHTUNG ZUM ZUSAMMENFÜHREN WENIGSTENS ZWEIER LEITUNGSABSCHNITTE BEI EINEM UNTERDRUCKWUNDBEHANDLUNGSSYSTEM**
CONNECTION DEVICE FOR BRINGING TOGETHER AT LEAST TWO LINE SECTIONS IN A VACUUM-BASED WOUND TREATMENT SYSTEM
DISPOSITIF DE LIAISON POUR RÉUNIR AU MOINS DEUX SECTIONS DE CONDUITES D'UN SYSTÈME DE THÉRAPIE PAR PRESSION NÉGATIVE SUR PLAIE

(30) Priorität: 13.08.2010 DE 102010034292
(43) Veröffentlichungstag der Anmeldung: 23.05.2012
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); HOFSTETTER, Jürgen, 89522 Heidenheim (DE)
(74) Vertreter: DREISS Patentanwälte PartG mbB
(86) Internationale Anmeldenummer: PCT/EP2011/062116
(87) Internationale Veröffentlichungsnummer: WO 2012/019865

(56) Entgegenhaltungen:
- EP-A2- 0 332 366
- WO-A1-01/34223
- WO-A1-2012/019865
- WO-A2-2007/106589
- GB-A- 2 402 724
- US-A- 4 931 049
- US-A1- 2003 014 022
- US-A1- 2005 046 184
- US-A1- 2007 156 104
- US-A1- 2008 011 368
- US-B1- 7 658 205

## Beschreibung

Die Erfindung betrifft ein System zum Zusammenführen wenigstens zweier Leitungsabschnitte bei einem Unterdruckwundbehandlungssystem, umfassend die beiden Leitungsabschnitte und eine Verbindungsvorrichtung, wobei die beiden Leitungsabschnitte in Bezug auf die Verbindungsvorrichtung wundseitig angeordnet sind und zu verschiedenen Wundanschlüssen bei einer Wunde oder zu Wundanschlüssen bei verschiedenen Wunden desselben Patienten führen, wobei die Verbindungsvorrichtung auf ihrer wundabgewandten Seite mit einem zu einer Unterdruck erzeugenden Einrichtung führenden dritten mehrlumigen Leitungsabschnitt verbindbar ist.

Die Anmelderin geht davon aus, dass derartige, typischerweise Y-förmige Verbindungsvorrichtungen zum Zusammenführen zweier Leitungsabschnitte zu einem dritten Leitungsabschnitt vorbekannt sind.

Hiervon ausgehend liegt der vorliegenden Erfindung die Aufgabe zugrunde, eine Verbindungsvorrichtung anzugeben, die es gestattet, eine mehrlumige Fluidkommunikation zu wenigstens zwei Wundanschlüssen zu erreichen, wobei hier der Begriff "Fluid" im weitesten Sinne verstanden werden soll und die Übermittlung von Flüssigkeiten oder Gasen oder die Erzeugung von Unterdruck erfasst sein soll.

Diese Aufgabe wird erfindungsgemäß durch ein System mit den Merkmalen des Anspruchs 1 gelöst.

Somit ist es möglich, zwei mehrlumig ausgebildete, von einem jeweiligen Wundanschluss kommende Leitungsabschnitte, die jeweils eine beispielsweise steckbare und insbesondere verrastbare Anschlussvorrichtung tragen, mit der einen Verbindungsvorrichtung beispielsweise durch Steckverbindung, Schraubverbindung oder dergleichen, zu koppeln. Im Inneren der Verbindungsvorrichtung werden dann einander entsprechende Lumen der Leitungsabschnitte zusammengeführt und in der Folge strömungsmäßig mit einem der mehreren Lumen des dritten zu der Unterdruck erzeugenden Einrichtung führenden Leitungsabschnitts verbunden. Auch diese Verbindung kann vorteilhafterweise mittels einer beispielsweise steckbaren und insbesondere verrastbaren Anschlussvorrichtung an dem dritten Leitungsabschnitt hergestellt werden.

Erfindungsgemäß wird sichergestellt, dass voneinander getrennte Lumen jedes der beiden wundseitigen Leitungsabschnitte auch im weiteren Verlauf getrennt voneinander bleiben. Dies macht die Verwendung der wenigstens zwei Lumen eines jeweiligen Leitungsabschnitts zu unterschiedlichen Zwecken überhaupt erst möglich. Es wird ein Lumen zum Anlegen von Unterdruck an eine Wunde und ein anderes Lumen als Spülkanal zum Zuführen eines gasförmigen oder flüssigen Spülmediums in Richtung auf die Wunde und/oder zu einem wundnahen Ausgangsbereich des erstgenannten Lumens verwendet.

Die genannte Verbindungsvorrichtung könnte grundsätzlich aus mehreren voneinander getrennten Teilen, insbesondere von mehreren Y-artigen Verbindungselementen gebildet sein, die mit den jeweiligen Anschlussvorrichtungen der Leitungsabschnitte zusammenwirken. In Weiterbildung der Erfindung wird jedoch vorgeschlagen, dass die Verbindungsvorrichtung von einem einstückig ausgebildeten Verbindungsstück gebildet ist, bei dem es sich insbesondere und vorzugsweise um ein Spritzgussteil handeln kann. Wenn vorstehend von einem einstückig ausgebildeten Verbindungsstück die Rede ist, so bedeutet dies jedoch nicht, dass keine zusätzlichen Dichtmittel, wie zum Beispiel O-Ringe oder dergleichen Dichthilfsmittel vorgesehen sein dürfen. Ein einstückig ausgebildetes Verbindungsstück im Sinne der vorliegenden Erfindung bedeutet vielmehr, dass das Verbindungsstück aus einem einzelnen Gehäuseteil gebildet ist.

Nach einer bevorzugten Ausführungsform der Erfindung sind im inneren der Verbindungsvorrichtung jeweils zwei wundseitige Lumen zu einem wundabgewandten Lumen zusammengeführt. Es wäre aber auch denkbar, dass die Verbindungsvorrichtung für mehr als zwei wundseitige Leitungsabschnitte, die ihrerseits zwei oder mehr Lumen aufweisen, ausgebildet ist.

Erfindungsgemäss wird vorgeschlagen, dass die jeweilige Zusammenführung der wenigstens zwei wundseitigen Lumen zu dem einen wundabgewandten Lumen jeweils in einer Ebene angeordnet ist. Dies gestattet eine verhältnismäßig einfache Konstruktion der Verbindungsvorrichtung.

Des weiteren erweist es sich als vorteilhaft, wenn die mehrlumigen Leitungsabschnitte sowie die Verbindungsvorrichtung und die jeweiligen Anschlussvorrichtungen an den Leitungsabschnitten eine Seite-an-Seite-Anordnung der Lumen aufweisen. Auch dies vereinfacht die Ausbildung der Anschlussvorrichtungen an den jeweiligen Leitungsabschnitten und der erfindungsgemäßen Verbindungsvorrichtung, da sich hierdurch die Konstruktion und die Herstellung der nach außen abdichtenden Kopplungsbereiche weniger komplex gestaltet.

Des weiteren erweist es sich als zweckmäßig, wenn die Verbindungsvorrichtung einerseits, insbesondere wundseitig, weibliche Aufnahmen für die jeweiligen Anschlussvorrichtungen und andererseits, insbesondere wundabgewandt, männliche Anschlüsse für die jeweiligen Anschlussvorrichtungen aufweist. Es wäre aber auch andersherum denkbar.

Wie eingangs bereits ausgeführt, erweist es sich als vorteilhaft, wenn von den voneinander getrennten Lumen der Leitungsabschnitte und der Verbindungsvorrichtung ein Lumen zum Anlegen von Unterdruck an eine Wunde und ein anderes Lumen als Spülkanal zum Zuführen eines gasförmigen oder flüssigen Spülmediums in Richtung auf die Wunde und/oder zu einem wundnahen Ausgangsbereich des erstgenannten Lumens ausgebildet ist.

Es erweist sich solchenfalls als vorteilhaft, wenn ein Lumen des wundabgewandten dritten Leitungsabschnitts mit einem gesteuert öffenbaren und verschließbaren Spülanschluss strömungsmäßig verbunden ist, um ein gasförmiges oder flüssiges Spülmedium in Richtung auf die Wunde und/oder zu einem wundnahen Ausgangsbereich eines Lumens der wundseitigen Leitungsabschnitte zu führen.

Weiter ist Gegenstand der vorliegenden Erfindung ein System mit einem auf der wundabgewandten Seite der Verbindungsvorrichtung vorgesehenen dritten mehrlumigen Leitungsabschnitt, von dem mindestens ein Lumen mit einer Unterdruck erzeugenden Einrichtung und wenigstens ein Lumen mit einem steuerbaren Anschluss zum Einleiten eines fluiden Spülmediums verbindbar ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Verbindungsvorrichtung. In der Zeichnung zeigt:

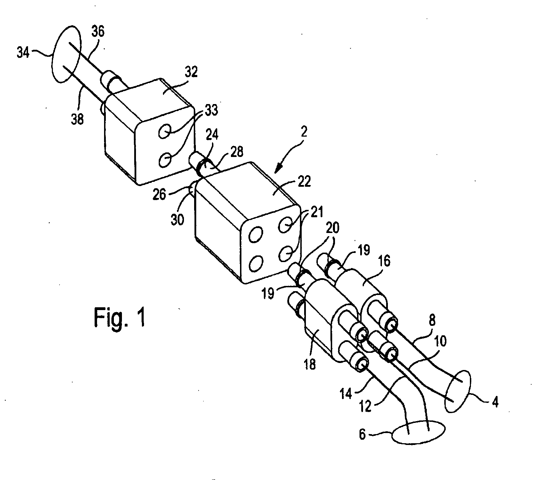

Figur 1 eine perspektivische Ansicht eines erfindungsgemäßen Systems mit wundseitigen und wundabgewandten Anschlussvorrichtungen bei lediglich angedeuteten mehrlumigen Leitungsabschnitten;
Figuren
   2a+2b zeigen eine Draufsicht und eine Seitenansicht der Komponenten nach Figur 1.

Die Figuren zeigen ein erfindungsgemäßes System mit einer Verbindungsvorrichtung 2 zum Zusammenführen zweier jeweils zweilumiger Leitungsabschnitte 4, 6, die lediglich schematisch angedeutet sind und zu einer oder mehreren Wunden das heißt zu im wesentlichen unterdruckdichten Wundabdeckungen bzw. Wundanschlüssen (nicht dargestellt) führen. Die Leitungsabschnitte 4, 6 weisen je zwei Lumen 8, 10 bzw. 12, 14 auf. Die Leitungsabschnitte 4, 6 bzw. deren Lumen 8, 10 und 12, 14 sind nach außen dicht mit Anschlussvorrichtungen 16, 18, beispielhaft nach Art von Steckadaptern, ausgestattet. Diese Anschlussvorrichtungen 16, 18, die im beispielhaft dargestellten Fall mit männlichen Anschlusssteckern 19 ausgestattet sind, können wiederum nach außen druckdicht mit der Verbindungsvorrichtung 2 in Strömungsverbindung gebracht werden. Im beispielhaft dargestellten Fall ist hierfür die Verbindungsvorrichtung 2 mit weiblichen Aufnahmen 21 für die männlichen Anschlussstecker 19 der Anschlussvorrichtungen 16, 18 ausgebildet. Zum dichtenden Abschluss weisen die Anschlussvorrichtungen 16, 18 an ihren Anschlusssteckern 19 außerdem Dichtmittel in Form von O-Ringen 20 auf.

Die Verbindungsvorrichtung 2 umfasst im beispielhaft dargestellten Fall einen vorzugsweise einstückig ausgebildeten Grundkörper 22, bei dem es sich wiederum beispielhaft und vorteilhaft um ein Spritzgussteil handeln kann.

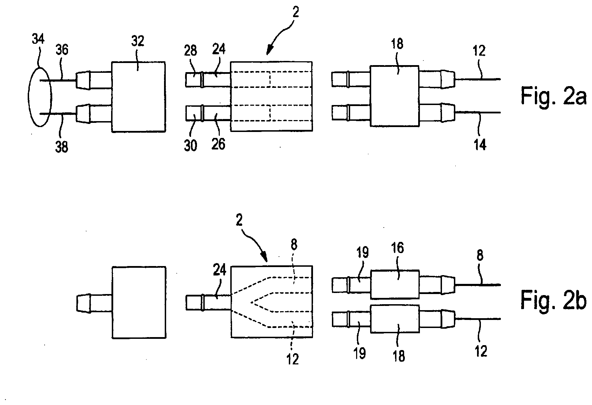

Auf nachfolgend noch näher zu beschreibende Weise sind im Inneren der Verbindungsvorrichtung 2 die Lumen 8, 12 und 10, 14 der Leitungsabschnitte 4, 6 zu je einem wundabgewandten Lumen 24, 26 zusammengeführt. Wie sich den Figuren 2a und 2b entnehmen lässt, geschieht dies dadurch, dass im Inneren der Verbindungsvorrichtung 2 die Lumen 8, 12 und 10, 14 der Leitungsabschnitte 4 bzw. 6 jeweils in einer Ebene zusammengeführt sind, also jeweils zwei Lumen zu einem Lumen. Auf ihrer wundabgewandten Seite verfügt die Verbindungsvorrichtung 2 über zwei je einlumige Ausgänge, die im beispielhaft dargestellten Fall als männliche Steckerteile 28, 30 ausgebildet sind und mit einer wundabgewandten Anschlussvorrichtung 32 mit weiblichen Aufnahmen 33 zusammenwirken. Die Anschlussvorrichtung 32 steht ihrerseits mit einem zweilumigen Leitungsabschnitt 34 mit Lumen 36, 38 in Strömungsverbindung und führt zu einer nicht dargestellten Unterdruck erzeugenden Einrichtung.

in besonders vorteilhafter Weise kommunizieren die Lumen 36, 38 des wundabgewandten Leitungsabschnitts 34 aber nicht beide mit einer Unterdruck erzeugenden Einrichtung, sondern eines der Lumen 36, 38 ist in vorteilhafter Weise als Spülkanal für die Zuführung eines fluiden, also gasförmigen oder flüssigen Spülmediums ausgebildet, welches bei Bedarf und/oder gesteuert in Abhängigkeit bestimmter Anforderungen in Richtung auf die Wunde freigegeben werden kann. Auf diese Weise kann, ausgehend von einer einzigen Unterdruck erzeugenden Vorrichtung und einer Spülsteuervorrichtung (beide nicht dargestellt) einerseits Unterdruck gleichzeitig über zwei voneinander getrennte Leitungsabschnitte 4, 6 an verschiedene Wundanschlüsse derselben Wunde oder Wundanschlüsse mehrerer Wunden desselben Patienten gebracht werden, und andererseits ist gleichzeitig eine Spülmöglichkeit zu jedem Wundanschluss gewährleistet.

## Patentansprüche

1. System zum Zusammenführen wenigstens zweier Leitungsabschnitte (4, 6) bei einem Unterdruckwundbehandlungssystem, umfassend die beiden Leitungsabschnitte (4, 6) und eine Verbindungsvorrichtung (2) und einen dritten mehrlumigen Leitungsabschnitt (34), wobei die beiden Leitungsabschnitte (4, 6) in Bezug auf die Verbindungsvorrichtung (2) wundseitig angeordnet sind und wobei die Verbindungsvorrichtung (2) auf ihrer wundabgewandten Seite mit dem zu einer Unterdruck erzeugenden Einrichtung führenden dritten mehrlumigen Leitungsabschnitt (34) verbindbar ist, **dadurch gekennzeichnet, dass** die wenigstens zwei Leitungsabschnitte (4, 6) mehrlumig ausgebildet sind und diese Leitungsabschnitte (4, 6) mit jeweils einer mehrlumigen mit der Verbindungsvorrichtung (2) in Strömungsverbindung gebrachten Anschlussvorrichtung (16, 18) verbunden sind und dass auch der dritte Leitungsabschnitt (34) eine Anschlussvorrichtung (32) zum Ankoppeln an die Verbindungsvorrichtung (2) aufweist, und dass voneinander getrennte Lumen (8, 10; 12, 14) der beiden wundseitigen Leitungsabschnitte (4, 6) auch innerhalb der Verbindungsvorrichtung (2) und innerhalb des dritten Leitungsabschnitts (34) voneinander getrennt bleiben, indem im Inneren der Verbindungsvorrichtung (2) jeweils wenigstens zwei wundseitige Lumen (8 und 12; 10 und 14) zu einem wundabgewandten Lumen (24, 26) zusammengeführt sind und dass die jeweilige Zusammenführung der wenigstens zwei wundseitigen Lumen (8; 12; 10, 14) zu dem einen wundabgewandten Lumen (24, 26) jeweils in einer Ebene angeordnet ist und wobei mindestens ein Lumen des dritten mehrlumigen Leitungsabschnitts (34) mit einer Unterdruck erzeugenden Einrichtung und wenigstens ein Lumen mit einem steuerbaren Anschluss zum Einleiten eines fluiden Spülmediums verhindbar ist.

2. System nach Anspruch 1, **dadurch gekennzeichnet, dass** die Verbindungsvorrichtung (2) von einem einstückig ausgebildeten Verbindungsstück (22) gebildet ist.

3. System nach Anspruch 1 oder 2, **dadurch gekennzeichnet, dass** die Verbindungsvorrichtung (2) als Spritzgußteil ausgebildet ist.

4. System nach einem oder mehreren der vorstehenden Ansprüche, **dadurch gekennzeichnet, dass** die mehrlumigen Leitungsabschnitte (4, 6, 34), die Verbindungsvorrichtung (2) und die Anschlussvorrichtungen (16, 18, 32) eine Seite-an-Seite-Anordnung der Lumen aufweisen.

5. System nach einem oder mehreren der vorstehenden Ansprüche, **dadurch gekennzeichnet, dass** die Verbindungsvorrichtung (2) einerseits, insbesondere wundseitig, weibliche Aufnahmen (21) für die jeweiligen Anschlussvorrichtungen (16, 18) und andererseits, insbesondere wundabgewandt, männliche Anschlüsse (28, 30) für die jeweiligen Anschlussvorrichtungen (32) aufweist.

6. System nach einem oder mehreren der vorstehenden Ansprüche, **dadurch gekennzeichnet, dass** von den voneinander getrennten Lumen (8, 10; 12, 14; 36, 38; 24, 26) der Leitungsabschnitte (4, 6, 32) und der Verbindungsvorrichtung (2) ein Lumen zum Anlegen von Unterdruck an eine Wunde und ein anderes Lumen als Spülkanal zum Zuführen eines gasförmigen oder flüssigen Spülmediums in Richtung auf die Wunde und/oder zu einem wundnahen Ausgangsbereich des erstgenannten Lumens ausgebildet ist.

7. System nach einem oder mehreren der vorstehenden Ansprüche, **dadurch gekennzeichnet, dass** ein Lumen des wundabgewandten dritten Leitungsabschnitts (34) mit einem gesteuert öffenbaren und verschließbaren Spülanschluss strömungsmäßig verbunden ist, um ein gasförmiges oder flüssiges Spülmedium in Richtung auf die Wunde und/oder zu einem wundnahen Ausgangsbereich eines Lumens der wundseitigen Leitungsabschnitte (4, 6) zu führen.

## Claims

1. System for merging at least two line sections (4, 6) in a vacuum wound treatment system, comprising the both line sections (4, 6) and a connection device (2) and a third multilumen line section (34), wherein the both line sections (4, 6) are arranged wound side in relation to the connection device (2), and wherein the connection device (2) is connectable on its side which faces away from the wound with the third multi-lumen line section (34) which leads to a vacuum generating device, **characterized in that** the at least two line sections (4, 6) are configured multi-lumen and these line sections (4, 6) each are coupled with a multi-lumen attachment device (16, 18) which is in flow communication with the connection device (2) and that the third line section (34) also has an attachment device (32) for coupling to the connection device (2), and **in that** lumens (8, 10; 12, 14) of the both wound side line sections (4, 6) which are separated from one another, also remain separated from one another within the connection device (2) and within the third line section (34), wherein in the interior of the connection device (2) respectively at least two wound-side lumens (8 and 12; 10 and 14) are merged into one lumen (24, 26) which faces away from the wound and that the respective merging of the at least two wound side lumens (8, 12; 10, 14) into the one lumen (24, 26) which faces away from the wound is arranged in a respective plane and wherein at least one lumen of the third multilumen line section (34) is connectable with a vacuum generating device and at least one lumen is connectable with a controllable connection for introducing a fluid rinsing medium.

2. System according to claim 1, **characterized in that** the connection device (2) is formed from a connection part (22) which is configured one-piece.

3. System according to claim 1 or 2 **characterized in that** the connection device (2) is configured as injection molding part.

4. System according to one or more of the preceding claims, **characterized in that** the multi-lumen line sections (4, 6, 34), the connection device (2) and the attachment devices (16, 18, 32) have a side-by-side arrangement of the lumens.

5. System according to one or more of the preceding claims, **characterized in that** the connection device (2) has on one side, in particular wound-side, female receptacles (21) for the respective attachment devices (16, 18) and on the other side, in particular facing away from the wound, male connections (28, 30) for the respective attachment devices (32).

6. System according to one or more of the preceding claims, **characterized in that** one lumen of the lumens (8, 10; 12, 14; 36, 38; 24, 26) of the line sections (4, 6, 32) and the connection device (2), which are separated from one another is configured for applying a negative pressure to a wound and another lumen is configured as rinsing channel for supplying a gaseous or liquid rinsing medium in the direction toward the wound and/or toward an outlet region of the first mentioned lumen near the wound.

7. System according to one or more of the preceding claims, **characterized in that** a lumen of the third line section (34) which faces away from the wound is connected in flow communication with a rinsing connection which can be opened and closed in a controlled manner in order to conduct a gaseous or liquid rinsing medium in the direction toward the wound and/or toward an outlet region of a lumen of the wound side line sections (4, 6) near the wound.

## Revendications

1. Système destiné à réunir aux moins deux sections de conduite (4, 6) dans un système de traitement des plaies par pression négative, comprenant les deux sections de conduite (4, 6) et un dispositif de liaison (2) et une troisième section de conduite (34) à lumens multiples, les deux sections de conduite (4, 6) étant disposées côté plaie par rapport audit dispositif de liaison (2), et ledit dispositif de liaison (2) pouvant être relié, de son côté opposé à la plaie, à une troisième section de conduite (34) à lumens multiples qui mène vers un dispositif générant une pression négative, **caractérisé en ce que** lesdites au moins deux sections de conduite (4, 6) sont réalisées à lumens multiples et ces sections de conduite (4, 6) sont reliées à respectivement un dispositif de raccordement (16, 18) à lumens multiples qui est mis en communication fluidique avec le dispositif de liaison (2), et que la troisième section de conduite (34) présente, elle aussi, un dispositif de raccordement (32) pour le couplage au dispositif de liaison (2) et que des lumens (8, 10; 12, 14) séparés les uns des autres des deux sections de conduite (4, 6) côté plaie restent séparés les uns des autres également à l'intérieur du dispositif de liaison (2) et à l'intérieur de la troisième section de conduite (34) par le fait que, à l'intérieur dudit dispositif de liaison (2), respectivement au moins deux lumens côté plaie (8 et 12; 10 et 14) sont réunis en un lumen (24, 26) opposé à la plaie et que la réunion respectif desdits au moins deux lumens côté plaie (8, 12; 10, 14) en ledit un lumen (24, 26) opposé à la plaie est disposé respectivement dans un plan et que au moins un lumen de la troisième section de conduit (34) peut être relié à un dispositif générant une pression négative et au moins un lumen peut être relié à un raccord commandable pour introduire un milieu fluide de rinçage.

2. Système selon la revendication 1, caractérisé en ce quele dispositif de liaison (2) est formé d'une pièce de liaison (22) réalisée en une seule pièce.

3. Système selon la revendication 1 ou 2, **caractérisé en ce que** le dispositif de liaison (2) est réalisé comme pièce moulée par injection.

4. Système selon l'une ou plusieurs des revendications précédentes, **caractérisé en ce que** les sections de conduite (4, 6, 34) à lumens multiples, le dispositif de liaison (2) et les dispositifs de raccordement (16, 18, 32) présentent une disposition côte à côte des lumens.

5. Système selon l'une ou plusieurs des revendications précédentes, **caractérisé en ce que** le dispositif de liaison (2) présente, d'un côté, en particulier côté plaie, des logements femelles (21) pour les dispositifs de raccordement (16, 18) respectifs et, de l'autre côté, en particulier opposé à la plaie, des raccords mâles (28, 30) pour les dispositifs de raccordement (32) respectifs.

6. Système selon l'une ou plusieurs des revendications précédentes, **caractérisé en ce que**, parmi les lumens (8, 10; 12, 14; 36, 38; 24, 26) séparés les uns des autres des sections de conduite (4, 6, 34) et du dispositif de liaison (2), un lumen est réalisé pour l'application d'une pression négative à une plaie et un autre lumen est réalisé comme canal de rinçage pour amener un milieu de rinçage gazeux ou liquide en direction de la plaie et/ou à une zone de sortie proche de la plaie, du lumen mentionné en premier.

7. Système selon l'une ou plusieurs des revendications précédentes, **caractérisé en ce qu'**un lumen de ladite troisième section de conduite (34) opposée à la plaie est relié fluidiquement à un raccord de rinçage apte à être ouvert et fermé de manière commandée, afin de mener un milieu de rinçage gazeux ou liquide en direction de la plaie et/ou vers une zone de sortie proche de la plaie, d'un lumen des sections de conduite (4, 6) côté plaie.
